# EUROPEAN PATENT SPECIFICATION

(11) **EP 1 793 750 B1**
(45) Date of publication and mention of the grant of the patent: **12.09.2012**
(21) Application number: 05761449.7
(22) Date of filing: 20.07.2005
(51) Int. Cl.: A61B 17/44

(54) **OBSTETRIC VACUUM EXTRACTOR**
VAKUUMEXTRAKTOR FÜR DIE GEBURTSHILFE
VENTOUSE OBSTETRIQUE

(30) Priority: 20.07.2004 GB 0416172
(43) Date of publication of application: 13.06.2007
(73) Proprietor: Monty Medical Limited, Redditch, Worcestershire B98 8BT (GB)
(72) Inventor: GEORGE, Samuel, Weybridge Surrey KT13 9SB (GB)
(74) Representative: Cummings, Sean Patrick
(86) International application number: PCT/GB2005/002863
(87) International publication number: WO 2006/008532

(56) References cited:
- EP-A- 0 797 958
- DE-A1- 2 739 589
- DE-A1- 19 629 624
- US-A- 3 794 044
- US-A- 5 810 840

## Description

This invention relates to the field of obstetrics and particularly to apparatus for assisting in the delivery of babies.

Assisted delivery may be recommended if a baby becomes distressed or fails to make adequate progress through the birth canal during labour, or if the mother is unable to push due to tiredness or a medical condition and so needs help to expel the baby. Assisted delivery usually implies the use of either forceps or a vacuum extractor known generically as a 'ventouse'. The terms 'ventouse' and 'vacuum extractor' will be used interchangeably in this specification.

Forceps typically have two intersecting metal parts with curved distal ends to cradle the baby's head. In use, the mother is placed on her back on a bed in the lithotomy position with her legs in stirrups. After being catheterised, she undergoes an episiotomy to enlarge the opening of her vagina so that the distal ends of the forceps can be put round the baby's head within her dilated cervix. Once the forceps are in place and locked, the attending physician pulls the proximal handle part of the forceps in time with the mother's contractions to help the baby progress through the birth canal.

In contrast, a ventouse comprises a vacuum cup communicating with a source of vacuum, such as a hand-held or foot-operated vacuum pump connected to the cup by a short tube. It is also possible to connect the cup to a suction line leading to a remote vacuum source such as a vacuum reservoir system built in to a hospital. In use, with the mother in the lithotomy position as aforesaid, the cup is inserted into the vagina and oriented to fit on top of a baby's head, whereupon the cup is fixed to the baby's head by suction as the attending physician draws air out of the cup through the tube or suction line, causing the cup to seal round its distal periphery to the baby's scalp. A handle or strap attached proximally to the cup then enables force to be applied to the cup to manipulate the baby's position, the physician pulling the handle or strap in time with the mother's contractions to help the baby progress through the birth canal. Where a handle is used and is rigid enough to transmit such force, the physician may also apply some torsion through the handle in an attempt to turn the baby. The tube through which air is drawn from the cup may form part of the handle or it may effect attachment of the handle to the cup.

The cup is typically made of rigid metal or semi-rigid plastics but may alternatively be made of substantially softer silicone plastics. Soft cups are less likely to damage a baby's head in use; conversely, relatively rigid cups are less likely to slip off and so have to be reattached. The type of cup used may depend on the baby's position. If the baby is in a position that makes delivery more difficult, then a metal cup or a semi-rigid plastics cup may be used in preference to a soft plastics cup because the more rigid types are less likely to become dislodged.

There is also a choice of cup sizes. As a rule, the wider the cup, the more securely the cup will be held to a baby's head under a given vacuum, as sub-atmospheric pressure is applied across a greater area of the cup. Occasionally, however, the cup comes off a baby's head in use and has to be reapplied. This happens especially if the baby's head is large or in a position where the cup does not fit well. Repeated slippage of the cup increases the probability of having to resort to forceps or a caesarean section.

A wide cup is also desirable because applying a given force over a greater area of the baby's head is less likely to injure the baby. For example, a baby often has a temporarily deformed head after use of a ventouse: in particular, the baby's head may be left with a lump called a chignon, and/or a blood blister, either of which may take some days to resolve.

Of course, there is a limit as to how large a cup can be before it becomes difficult to insert into the mother's vagina, risking discomfort or injury to her. In general, if a ventouse cup is small and soft so that it can be deformed to fit into the vagina, there is a fair chance that the mother will not require an episiotomy; conversely, the larger and more rigid the cup, the more likely it is that an episiotomy will be needed. There is therefore a trade-off between the efficacy of the cup and the comfort of the mother.

It is generally accepted that forceps present a greater risk of injury to the mother and baby than the use of a ventouse. For example, forceps deliveries may bruise the baby and may disturb the mother's bladder and bowel functions. Certainly, women have reported that forceps delivery causes greater discomfort both during and after birth than ventouse delivery. It is also crucial for the physician to know the orientation of the baby's head when using forceps, and forceps can be difficult to lock once in position. In contrast, vacuum extraction can be performed without exact knowledge of the orientation of the head and before the cervix is completely dilated because the suction cup takes no additional space around the baby's head. This can help a very distressed baby to be born quickly. Also, it is difficult for a physician using a ventouse to apply excessive force to a baby's head as the cup will tend to slip off in that event. Consequently, many physicians prefer using a ventouse to forceps for assisted deliveries.

Whilst the idea of using a suction cup upon the foetal head dates back to the eighteenth century, the modem vacuum extractor was invented by Malmström in 1954 and there has been considerable patent activity in that field ever since. For example, US Patent Nos. 5,019,086 and 5,810,840, from which the two-part form of claim 1 is based, disclose vacuum extractors having a rigid cup, an elongated stem and flexible means between the cup and the stem allowing the cup to be folded into an insertion position substantially parallel to the stem thereby facilitating insertion of the cup into the birth canal. However, the diameter of the cup itself cannot be reduced in this manner. By contrast, US Patent Nos. 5,224,947 and US 5,569,265 are examples of flexible cups made of a soft and resilient material, the latter being in the form of a bonnet that can be rolled over the baby's cranium. More recently, an International patent application published as WO99/58071 disclosed a hand-held vacuum extractor that combines a pump and a handle into a single hand-held unit connected to a rigid vacuum cup by a tube. Advantageously, a physician can control vacuum using the pump and apply traction via the tube using one hand. This is the basis of a commercially-successful vacuum extractor known among obstetricians as the 'Kiwi' device, which employs a cup of standard 5 cm diameter. However, the Kiwi device is not suitable for turning the baby or for mid-cavity use.

Despite these efforts to improve vacuum extractors, the physician is still faced with a choice of cup sizes or cup materials that have advantages in some situations but disadvantages in others. For example, the use of a large and rigid cup may be desirable for secure fixing to the baby's head but it may also be unnecessarily painful and distressing for the mother. Conversely, a flexible and small cup may be more comfortable for the mother, at least initially, but its weak fixing may lead to so many reapplications to the baby's head that eventually it becomes distressing for the mother and, indeed, hampers delivery. Moreover, a flexible cup can be surprisingly difficult to deform into a small diameter for insertion because squeezing together its opposed sides on one axis will cause the cup to widen perpendicularly to that axis.

Whilst it may be superficially desirable to have a choice of cups available in the delivery room, it is inefficient to stock different cup sizes where some of those cups may be used only rarely. There is also an element of guesswork as to which cup to select. In the dynamic environment of the delivery room, it is impractical and unfair on a mother to adopt trial and error, trying a multitude of cups to see which one is best for the delivery in question. Changing the cup size involves removing one cup from the baby's head and then unwrapping and reapplying another cup after, possibly, disconnecting the original cup from a source of vacuum and connecting the next cup to that source. So, once a choice of cup is made, the physician feels reluctant to make another choice even if the first choice turns out not to be ideal. Put another way, once the physician has chosen a cup of a given rigidity, size and shape to enjoy its desired advantages, the physician, the mother and the baby must also suffer any disadvantages that are inherent in that cup.

US Patent No. 6,090,041 discloses a vacuum-actuated surgical retractor for retracting tissue (e.g. an organ) of a patient during surgery. Whilst it does not disclose an obstetric vacuum extractor, it proposes a retractor having a flexible end-piece that can convert passively between a convex shape and a concave shape upon encountering tissue. The overall diameter of the retractor head cannot be reduced for the purpose of insertion unless used with a catheter for endoscopic procedures - in which case the distal part of the head is not convex when it is of reduced diameter and being inserted.

Against this background, the invention resides in a head for an obstetric vacuum extractor, the head having a convex distal part. In relation to US Patent No. 5,810,840, the invention is characterised in that the head is compressible laterally by a physician's fingers for insertion into a mother's vagina, and in that the shape of the convex distal part is reconfigurable, after insertion, into a concave suction cup for engagement with a baby's head, whereby force may be applied to the baby's head via the cup to aid delivery of the baby. The extractor head of the invention is optimally flexible for insertion and is optimally shaped for insertion.

The head preferably comprises a hollow flexible body such as a ball, in which case it is advantageous if the wall thickness of the hollow body increases proximally. This ensures reliable deformation into the desired cup shape. Nevertheless, there may be a relatively rigid distal cap, which cap is preferably convex and surmounts the distal end of the body. The cap helps to ensure reliable airflow when the cup is evacuated in use, for which purpose a plurality of distributed air channels are preferably disposed around the cap. It is also preferred that the cap is spaced from the body to permit air to flow under the cap. The head suitably comprises at least one air extraction channel for evacuating the suction cup which communicates with the air channels defined by the cap; in an elegantly simple arrangement, the air extraction channel includes the interior of the hollow body and a distal opening of the hollow body under the cap.

It is advantageous for the head to include reconfiguration means acting upon the distal part to define the cup, for example tensile means such as a chain extending from the distal end of the distal part to an actuator located proximally in relation to the head. The distal end of the tensile means may then be anchored to the cap.

The invention extends to an obstetric vacuum extractor having the head of the invention as defined above. The extractor suitably includes means for reconfiguring or deforming the distal part of the head, for example an actuator in a proximal handle part acting on tensile means extending distally to the head. The actuator preferably comprises a plunger movable proximally with respect to an outer tube defining a handle grip, and the plunger advantageously defines an internal air channel communicating with the head for evacuating the suction cup.

Means are preferably provided to hold the cup in a desired size and shape, for example by locking the plunger with respect to the outer tube. To that end, the plunger may include an array of longitudinally-spaced male locking formations selectively engageable with female locking formations on the outer tube. Elegantly, the selected locking formations may be engaged simply by turning the plunger within the outer tube.

The extractor may also include suction means communicating with the head for evacuating the suction cup. For example, the suction means can communicate with the head via the internal air channel of the plunger. Whilst the suction means can take many forms, preferred embodiments employ a syringe. Advantageously, the syringe has a one-way valve that opens to permit distal movement of a piston while closing upon proximal movement of the piston to draw air from the head. The syringe has an inner plunger carrying the piston within a barrel and may further includes means for locking the inner plunger with respect to the barrel. Like the aforementioned locking means, the inner plunger may include an array of longitudinally-spaced male locking formations selectively engageable with female locking formations on the barrel, for example by turning the inner plunger within the barrel. In a particularly elegant arrangement, the barrel of the syringe also serves as the plunger of the actuator.

The extractor of the invention preferably includes an integral suction indicator for indicating the degree of suction applied to the head. Whilst other arrangements are possible, the suction indicator may for example comprise a diaphragm responsive by deflection to the suction applied by the suction means.

In order that this invention may be more readily understood, reference will now be made, by way of example, to the accompanying drawings. It should be noted that dimensions mentioned in the following description are for illustration only and do not limit the broad scope of the invention. In the drawings:
Figure 1 is a part-sectioned side view of a vacuum extractor in accordance with a preferred embodiment of the invention, showing a distal head part of the extractor in an extended configuration ready for insertion upon use;
Figure 2 corresponds to Figure 1 and shows the distal head part of the extractor in a collapsed configuration defining a cup for attachment to a baby's cranium;
Figure 3 is an enlarged detail perspective view of a ball defining the distal head part of the vacuum extractor of Figures 1 and 2;
Figure 4 corresponds to Figure 3 but shows how the ball is surmounted by a cap;
Figure 5 is a perspective detail view of the cap visible in Figure 4, showing how a chain is connected to the underside of the cap;
Figure 6 is a sectional side view of the ball and cap of Figure 5, showing how the wall thickness of the ball varies;
Figure 7 is a sectional side view of the ball and cap of Figure 5 in various stages of deformation between the extended and collapsed configurations;
Figure 8 is a part-sectioned side view showing the vacuum extractor of Figure 1 in its extended configuration and connected to a syringe for generating vacuum;
Figure 9 corresponds to Figure 8 but shows the vacuum extractor with its distal part in the collapsed configuration of Figure 2;
Figure 10 is a part-sectioned side view of a second embodiment of the invention;
Figure 11 is a sectional side view of an outer tube being part of the embodiment of Figure 10;
Figure 12 is a sectional side view of a syringe barrel being part of the embodiment of Figure 10;
Figure 13 is a sectional side view of an inner plunger being part of the embodiment of Figure 10;
Figure 14 is an enlarged detail perspective view showing how the outer tube, syringe barrel and inner plunger of Figures 11, 12 and 13 fit together and interact in the embodiment of Figure 10;
Figures 15a to 15f are a sequence of schematic sectional side views showing the second embodiment in use; and
Figure 16 is part-sectioned side view of a third embodiment of the invention.

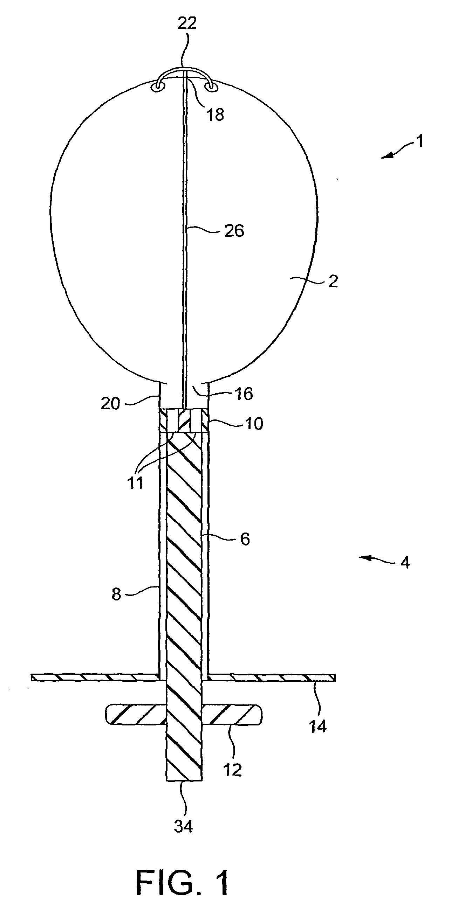

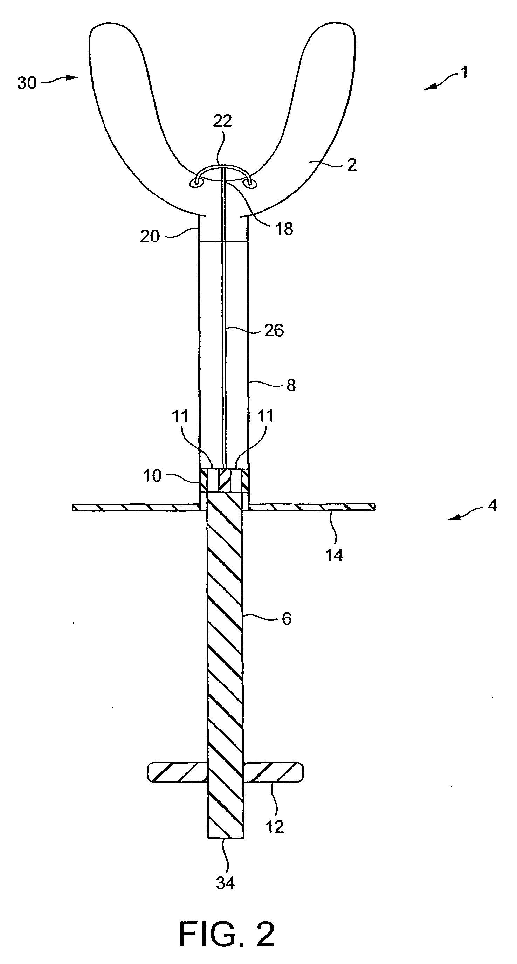

Referring firstly to Figures 1 and 2 of the drawings, an obstetric vacuum extractor 1 comprises an ovoid, hollow ball 2 of resiliently flexible moulded plastics defining a head joined to an elongate rigid tubular handle 4, which may also be of plastics. In its rest state, the ball 2 is approximately 8 cm in width and 9 cm in length. The handle 4 is adapted to configure the ball 2 for engagement with a baby's head and to apply force to the baby's head via the ball 2 once so engaged.

The handle 4 comprises a hollow tubular plunger 6 being a sliding fit within an outer tube 8, for which purpose the distal end of the plunger 6 terminates in a piston 10 that fits snugly within the outer tube 8. The proximal ends of the plunger 6 and the outer tube 8 terminate in transversely-extending grips 12, 14 to facilitate a physician's grip upon the extractor.

The ball 2 has openings 16, 18 at its distal and proximal ends. The proximal end of the ball 2 has a sleeve 20 attached to the distal end of the handle 4 such that the proximal opening 16 of the ball 2 aligns with the outer tube 8 of the handle 4, with the sleeve 20 being sealed around its periphery to the distal end of the outer tube 8. Conversely, the proximal opening 16 of the ball 2 communicates with the interior of the plunger 6 via openings 11 in the piston 10.

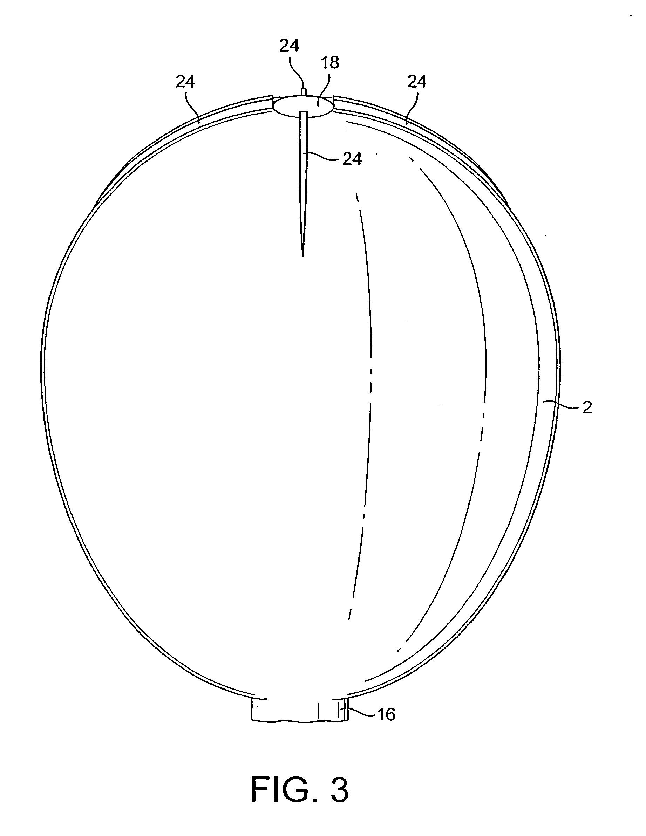

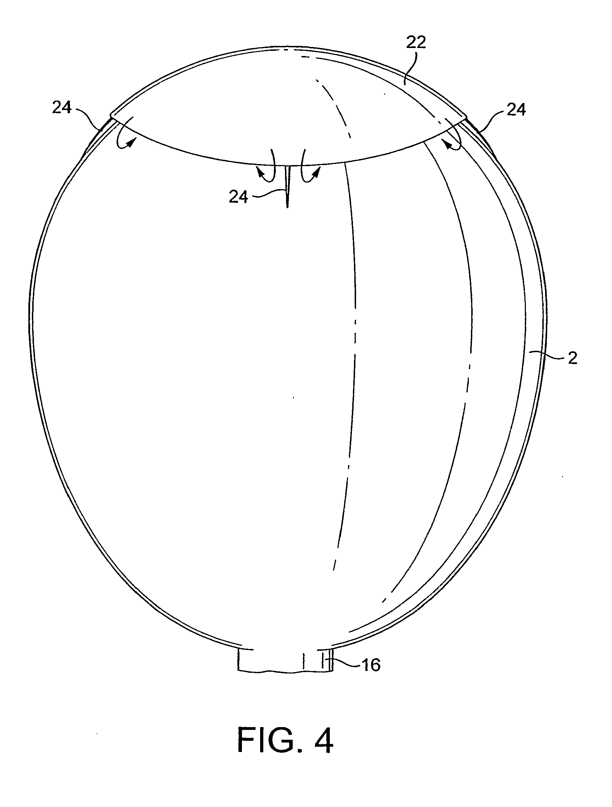

The distal opening 18 of the ball 2 is covered by an external convex rigid cap 22 about 3 cm in diameter, but in a manner that allows air to flow through the distal opening 18 from outside the cap 22 to within the ball 2. This is achieved by the arrangement shown in Figures 3 and 4. Figure 3 shows how four equi-spaced ridges 24 each about 3 cm in length radiate from the distal opening 18 across the outer surface of the ball 2, tapering outwardly with respect to the opening and proximally with respect to the distal end of the ball 2. Figure 4 shows how the cap 22 is supported by the ridges 24 while being held clear from the outer surface of the ball 2 because the ridges 24 are longer than the radius of the circular cap 22. This allows air to flow around the edge of the cap 22 as shown by the arrows, under the cap 22 and through the distal opening 18.

A chain 26 extends between the distal end of the plunger 6 and the underside of the cap 22, thus extending through the distal 18 and proximal 16 openings of the ball 2 to traverse the hollow interior of the ball 2 along its central longitudinal axis. The chain 26 becomes taut in use and may be taut at the outset, but there is preferably some slack so that the ball 2 can extend distally upon being squeezed laterally for insertion in use.

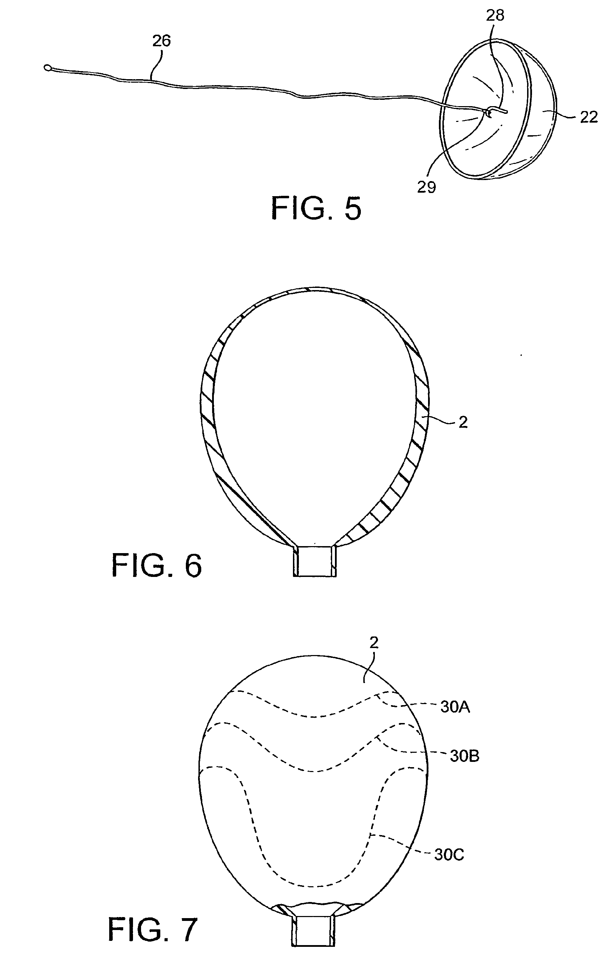

The attachment of the chain 26 to the cap 22 is best shown in the detail view of Figure 5, from which it will be noted that a hook 28 on the underside of the cap 22 engages with a link 29 at the distal end of the chain 26. It will be appreciated that tensile means other than a chain, for example a cord of plastics or wire, may be employed as an alternative. It is also possible to use anchorages other than a hook 28, for example bonding or welding the tensile means to the cap 22.

Reverting to Figures 1 and 2, it will be apparent that pulling the plunger 6 proximally with respect to the outer tube 8, as in Figure 2, pulls the chain 26. Consequently, the cap 22 attached to the chain 26 causes the ball 2 to collapse under external axial pressure applied by, and between, the cap 22 and the distal end of the handle 4. During this movement, the formerly convex distal part of the ball 2 inverts, becoming concave to define a cup 30. The cross-section of the cup 30 is akin to a sine wave.

The invention benefits from the Inventor's insight that by inverting a hollow single-walled convex structure to create a twin-walled concave structure, the stiffness of the structure can be increased. So, for the first time, the invention provides a vacuum extractor cup 30 whose stiffness can be varied in use. The ball 2 is optimally flexible for insertion, being easily compressible laterally by the physician's fingers, and indeed is optimally shaped for insertion; yet, when the ball 2 is transformed into a cup 30 upon encountering the baby's head, the cup 30 is optimally stiff for reliable attachment to the baby's head.

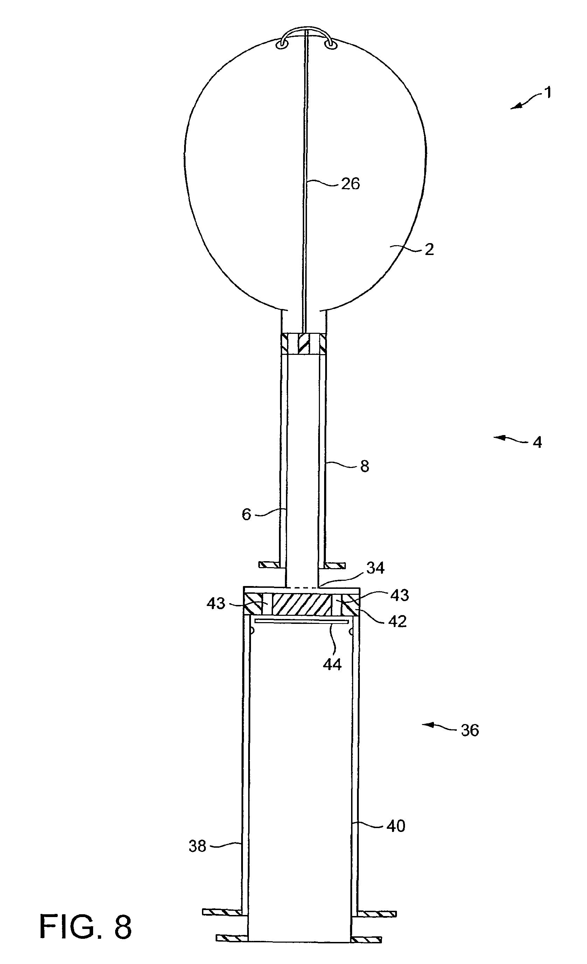

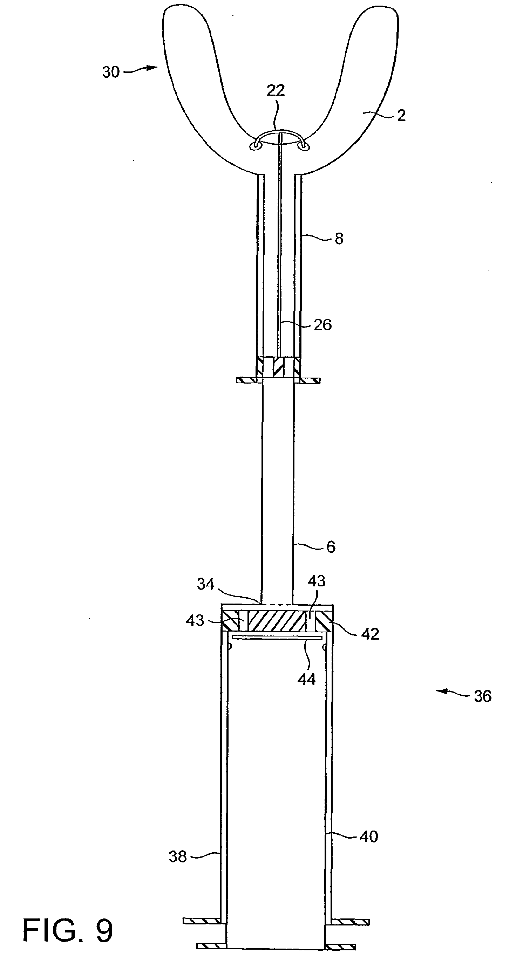

When the cup 30 shown in Figure 2 is engaged with a baby's head such that the peripheral lip of the cup 30 seals to the baby's scalp, the interior of the cup 30 is evacuated to grip the baby's head. This is achieved by connecting the proximal end 34 of the tubular plunger 6 to a vacuum source (not shown). Air evacuated from the cup 30 passes under the cap 22, through the distal 18 and proximal 16 openings of the cup 30, through the openings 11 in the piston 10, and through the plunger 6. The vacuum source can be of any suitable type, such as a wall-mounted or free-standing suction system, a foot-operated pump or a hand-held pump akin to that of the aforementioned Kiwi device. It is also possible to use a syringe arrangement as will be described later with reference to Figures 8 and 9.

The cap arrangement shown in Figures 3 and 4 is of importance in enabling and maintaining airflow out of the cup 30 in use. Air can flow under the cap 22 all round its edge, except obviously the points coinciding with the ridges 24. The effect is of multiple distributed air channels which ensures that the baby's scalp cannot block all airflow out of the cup 30. The convexity of the cap 22 and therefore its protrusion into the cup 30 also helps to keep the baby's scalp clear of the air channels.

Moving on now to Figure 6, this shows how the wall thickness of the hollow ball 2 preferably increases proximally while tapering distally. The effect is one of differential stiffness, the proximal part of the ball 2 being stiffer than its distal part. Thus, when the ball 2 is squeezed longitudinally by being compressed between the cap 22 and the distal end of the handle 4, the distal part collapses within the proximal part to define the cup 30. If the proximal part collapsed instead, a cup 30 would not be formed; similarly, if the stiffness of the ball 2 was uniform, collapse would be unpredictable meaning that even if a cup 30 was formed, it could lack the necessary planar peripheral lip.

Figure 7 shows that it is not necessary to collapse the ball 2 completely to define a usable cup 30. The plunger 6 and hence the chain 26 can be pulled to varying extents, from a small cup 30A created by a short pull of the plunger 6, through an intermediate cup 30B created by a longer pull, to a large cup 30C created by a yet longer pull. Thus, the invention also provides, for the first time, a vacuum extractor cup 30 whose diameter can be varied in use, responding freely to the changing circumstances of delivery. Indeed, cup 30 sizes could be anything from 3 cm to 8 cm in diameter. There is no longer a need to have a set of differently-sized cups 30 or to employ trial-and-error in their selection. Also, the diameter of the cup 30 can be increased to the extent permitted by the exposed area of the baby's head: as the cervix dilates, more of the scalp will be exposed and the area of the cup 30 can be expanded under continued suction to cover that exposed area. It is even possible to select a small cup 30 size for ease of insertion into the vagina and then to expand the cup 30 upon encountering the baby's head. So, it is not essential that the distal part of the ball 2 must be wholly convex for insertion.

As mentioned above, Figures 8 and 9 show a syringe arrangement 36 for applying suction to the proximal end 34 of the plunger 6 incorporated in the handle 4 of the device of the preceding Figures. Figure 8 shows the vacuum extractor 1 with the distal part of the ball 2 in its extended configuration as in Figure 1, and Figure 9 shows the vacuum extractor 1 with the distal part of its ball 2 in the collapsed configuration of Figure 2. Both drawings show that the syringe has a barrel 38 whose distal end is attached to and communicates with the hollow plunger 6 of the handle 4. So, pulling the barrel 38 of the syringe proximally as in Figure 9 also pulls the plunger 6 of the handle 4 to collapse the ball 2 into a cup 30 for engagement with a baby's head. The syringe is then operated to draw air through the plunger 6 of the handle 4 to evacuate the space between the cup 30 and the baby's head as aforesaid.

More specifically, a syringe plunger 40 within the barrel 38 of the syringe terminates distally in a piston 42 being a close sliding fit within the barrel 38. The piston 42 includes openings 43 closed by a diaphragm 44 disposed proximally with respect to the openings 43 to create a one-way valve. Both Figure 8 and Figure 9 show the piston 42 advanced to the distal end of the barrel, the valve opening to permit distal movement of the syringe plunger 40 into that position by allowing air to flow through the openings 43 in the piston 42. When the syringe plunger 40 is withdrawn proximally with respect to the barrel 38, the valve closes so that air is drawn from the plunger 6 of the handle 4.

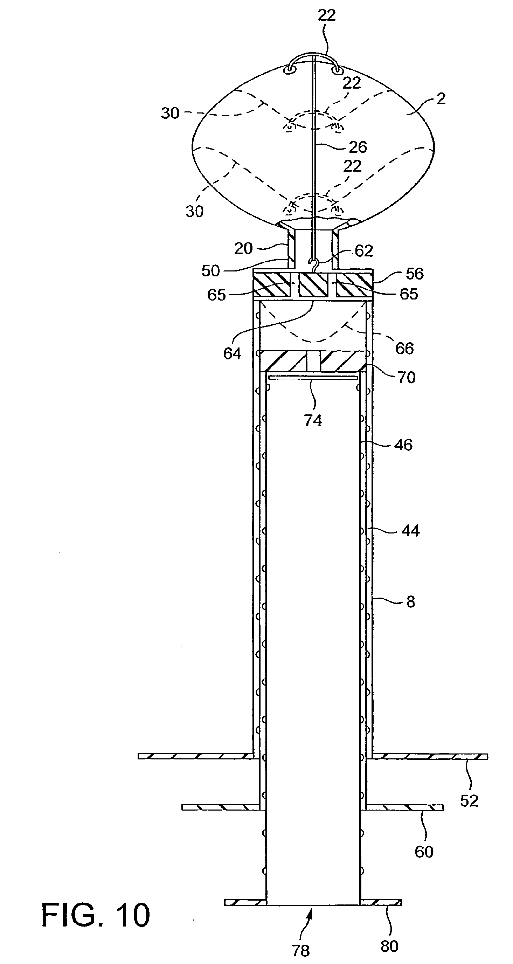

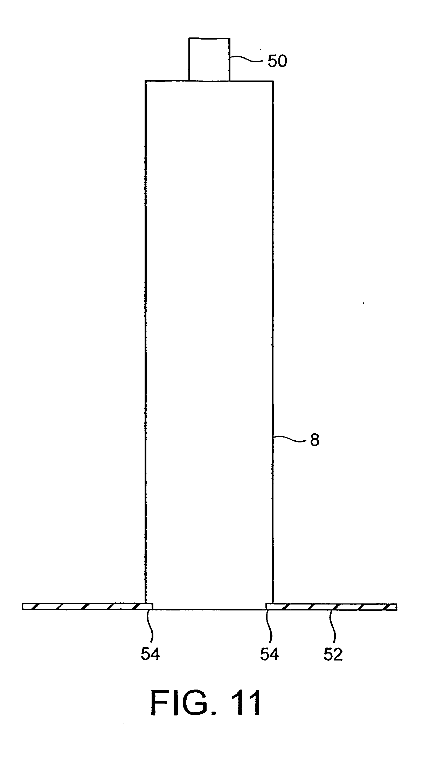

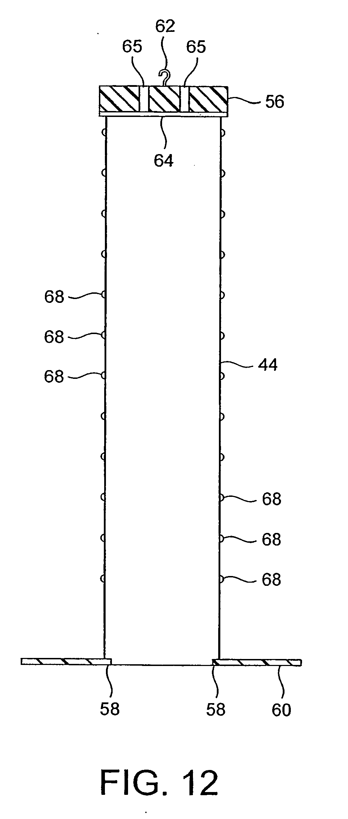

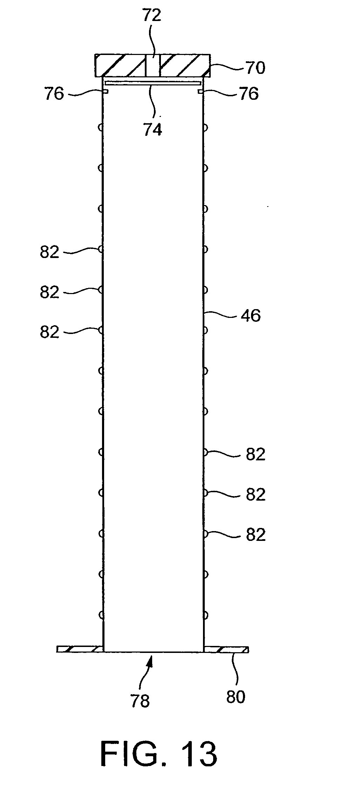

Figures 10 to 14 illustrate a second embodiment of the invention that incorporates various features described above in a more compact format and includes other features designed to facilitate operation of the device. A syringe barrel 44 and an inner plunger 46 fit within an outer tube 8 and move relative to each other in telescopic manner in use to collapse the ball 2 into a cup 30 and then to evacuate the space between the cup 30 and a baby's head. The syringe barrel 44 itself serves as a plunger.

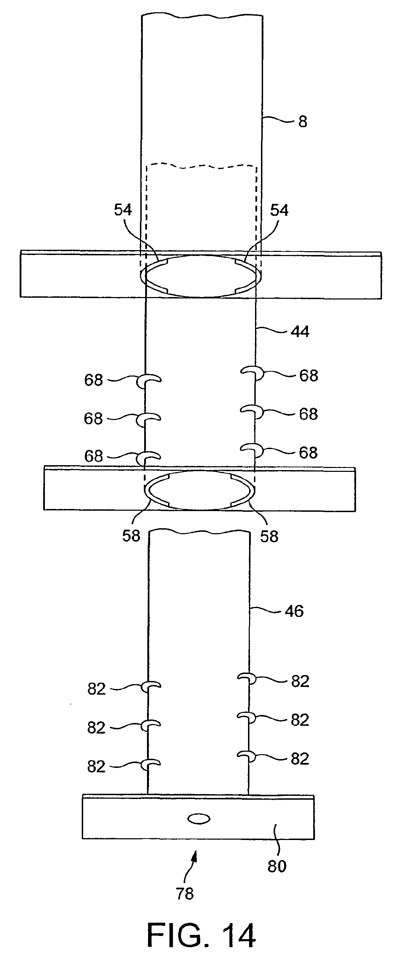

Referring firstly to Figures 10, 11 and 14, a graduated outer tube 8 terminates distally in a nozzle 50 and proximally in an open end having a flange 52. As Figure 10 shows, the nozzle 50 is attached to and communicates with a sleeve 20 defining the proximal opening 16 of the ball 2. Opposed constrictions 54 face inwardly at the open end of the outer tube 8. As best shown in Figure 14, each constriction is a female ridge extending just under a quarter of the circumference of the outer tube 8.

Figures 10, 12 and 14 show the syringe barrel 44 which is received in snug sliding manner within the outer tube 8. The syringe barrel 44 has a blind distal end terminating in a piston 56 that makes a sliding seal within the outer tube 8. An open proximal end has a flange 60 and opposed inwardly-facing constrictions 58 akin to those of the outer tube 8. The piston 56 carries a hook 62 for attachment to a chain 26 leading, as described previously, to the cap 22 that surmounts the ball 2. It will therefore be apparent that pulling the syringe barrel 44 proximally with respect to the outer tube 8 pulls the chain 26 which in turn collapses the ball 2 into a cup 30.

A thin, very flexible elastic transverse diaphragm 64 disposed proximally with respect to the piston 56 closes the distal end of the syringe barrel 44. The piston 56 is penetrated by holes 65 that expose the distal side of the diaphragm 64 to the air pressure experienced within the nozzle 50 of the outer tube 8, which equates to the pressure within the ball 2 and hence also between the cup 30 and a baby's head in use. When pressure on the distal side of the diaphragm 64 exceeds that on the proximal side of the diaphragm 64, the diaphragm 64 stretches and bulges proximally to an extent that can be measured against a graduated scale on the outer tube 8 as an indication of the pressure between the cup 30 and the baby's head. This deflection is shown schematically by the dashed lines 66 in Figure 10.

An array of longitudinally-spaced male ridges 68 extends circumferentially in opposed pairs along the exterior of the syringe barrel 44. As best shown in Figure 14, each ridge extends just under a quarter of the circumference of the syringe barrel 44 such that the ridges 68 can pass freely through the gap between the constrictions 54 of the outer tube 8. Nevertheless, an opposed pair of ridges 68 can be engaged with the constrictions 54 by means of a quarter-turn of the syringe barrel 44 about the central longitudinal axis of the outer tube 8. In this way, the syringe barrel 44 can be locked to the outer tube 8 at a position corresponding to a desired cup diameter. Indeed, graduations on the syringe barrel 44 and the outer tube 8 can be used to determine a desired cup diameter with reference to the relative positions of the syringe barrel 44 and the outer tube 8.

Figures 10, 13 and 14 show the inner plunger 46 which is received in snug sliding manner within the syringe barrel 44. The distal end of the inner plunger 46 terminates in a piston 70 that makes a sliding seal within the syringe barrel 44. The piston 70 is penetrated by a hole 72 closed by a diaphragm 74 disposed proximally with respect to the hole 72 to create a one-way valve akin to that of the syringe shown in Figures 8 and 9. The diaphragm 74 is held in place by an inwardly-facing circumferential flange 76 disposed proximally with respect to the diaphragm 74. Figure 10 shows the piston 70 advanced to the distal end of the syringe barrel 44, the valve opening to permit distal movement of the inner plunger 46 into that position by allowing air to flow through the hole 72 in the piston 70. When the inner plunger 46 is withdrawn proximally with respect to the syringe barrel 44, the valve closes so that air is drawn from the collapsed ball 2 and hence from the cup 30 defined by the ball 2. More specifically, suction is applied to the diaphragm 64 which deflects, indicating the degree of suction, and by virtue of that deflection applies suction in turn to the nozzle 50 of the outer tube 8. It will be evident to the skilled reader that the diaphragm 64 could be replaced by a piston acting against bias means such as a spring to transmit and indicate suction in similar manner.

An open proximal end 78 of the inner plunger 46 has a flange 80 akin to those of the outer tube 8 and the syringe barrel 44. Like the syringe barrel 44, the inner plunger 46 has an array of longitudinally-spaced male ridges 82 that extend circumferentially in opposed pairs along the exterior of the inner plunger 46. Again, as best shown in Figure 14, each ridge extends just under a quarter of the circumference of the inner plunger 46 such that the ridges 82 can pass freely through the gap between the constrictions 58 of the syringe barrel 44. Nevertheless, an opposed pair of ridges 82 can be engaged with the constrictions 58 of the syringe barrel 44 by means of a quarter-turn of the inner plunger 46 about the central longitudinal axis of the syringe barrel 44. In this way, the inner plunger 46 can be locked to the syringe barrel 44 at a position corresponding to a desired degree of suction. Indeed, graduations on the inner plunger 46 and the syringe barrel 44 can be used to determine a desired degree of suction with reference to the relative positions of the inner plunger 46 and the syringe barrel 44.

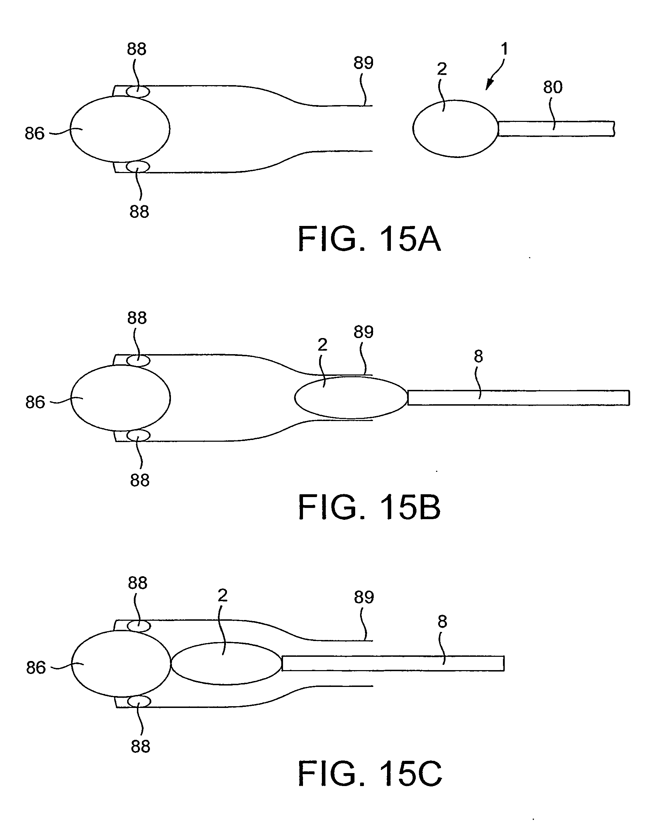

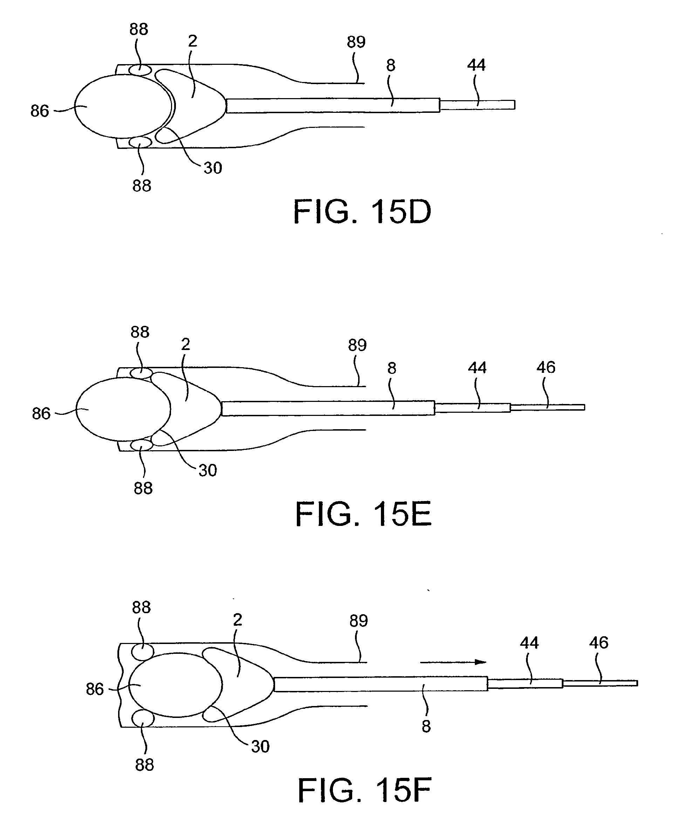

Figures 15a to 15f show the second embodiment, schematically, in use during delivery of a baby. A baby's head 86 is shown on the left within the mother's dilated cervix 88, the mother's vagina extending to the right to the vaginal opening 89. Figure 15a shows the extractor 1 with the ball 2 in a rest position. Figure 15b shows the extractor with the ball 2 laterally compressed for insertion into the vaginal opening 89 and Figure 15c shows the ball 2 encountering the baby's head 86. At this stage, the attending physician pulls the syringe barrel 44 to transform the convex distal part of the ball 2 into a concave cup 30, as shown in Figure 15d. The physician then pulls the inner plunger 46 to apply suction to the cup 30, pulling the cup 30 snugly against the baby's head 86 as shown in Figure 15e. Finally, the physician pulls and optionally also applies torsion to the extractor to pull the baby's head 86 through the cervix 88 as shown in Figure 15f.

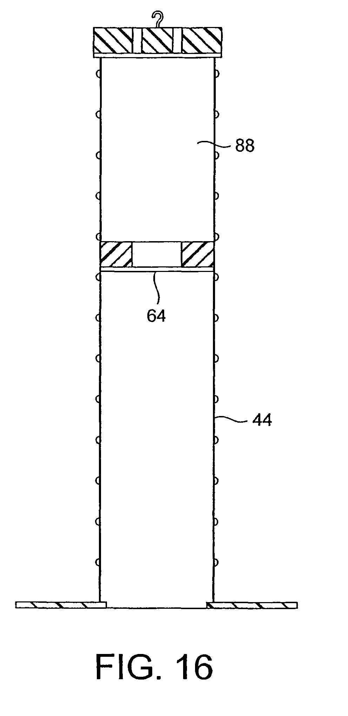

Finally, Figure 16 shows a syringe barrel 44 for use in a third embodiment of the invention derived from the second embodiment described above. Figure 16 shows how a reservoir 88 may be created in the distal part of the syringe barrel 44 by relocating the elastic membrane 64 proximally to a central location with respect to the length of the syringe barrel 44.

The invention may be embodied in many different forms and so is merely exemplified by the foregoing specific description. Reference should therefore be made to the appended claims rather than the foregoing specific description to determine the scope of the invention.

## Claims

1. A head (2) for an obstetric vacuum extractor (1), the head having a convex distal part, **characterised in that**:
the head is compressible laterally by a physician's fingers for insertion into a mother's vagina; and
the shape of the convex distal part is reconfigurable, after insertion, into a concave suction cup (30) for engagement with a baby's head, whereby force may be applied to the baby's head via the cup to aid delivery of the baby.

2. The head of Claim 1, comprising a hollow flexible body (2).

3. The head of Claim 2, wherein the wall thickness of the hollow body increases proximally.

4. The head of Claim 2 or Claim 3, comprising a relatively rigid distal cap (22).

5. The head of Claim 4, wherein the cap (22) is convex.

6. The head of Claim 4 or Claim 5, wherein a plurality of distributed air channels are disposed around the cap (22).

7. The head of any of Claims 4 to 6, wherein the cap (22) is spaced from the body (2) to permit air to flow under the cap.

8. The head of any of Claims 4 to 7, wherein the cap (22) surmounts the distal end of the body (2).

9. The head of any preceding Claim, comprising at least one air extraction channel (18) for evacuating the suction cup (30).

10. The head of Claim 9 when appendant to Claim 2, wherein the air extraction channel (18) includes the interior of the hollow body (2).

11. The head of Claim 9 or Claim 10 when appendant to Claim 6, wherein the air extraction channel (18) communicates with the air channels defined by the cap (22).

12. The head of Claim 11, wherein the air extraction channel (18) includes a distal opening of the hollow body (2) under the cap (22).

13. The head of any preceding Claim, including reconfiguration means (6, 26) for acting upon the distal part to define the cup (30).

14. The head of Claim 13, comprising tensile means (26) extending from the distal end of the distal part to an actuator (6) located proximally in relation to the head (2).

15. The head of Claim 14 when appendant to any of Claims 4 to 8, wherein the distal end of the tensile means (26) is anchored to the cap (22).

16. An obstetric vacuum extractor (1) having a head (2) as defined in any preceding Claim.

17. The extractor of Claim 16, further including means (6, 26) for reconfiguring or deforming the distal part of the head (2).

18. The extractor of Claim 17, wherein an actuator (6, 8) in a proximal handle part acts on tensile means (26) extending distally to the head (2).

19. The extractor of Claim 18, wherein the actuator (6, 8) comprises a plunger (6) movable proximally with respect to an outer tube (8) defining a handle grip.

20. The extractor of Claim 19, wherein the plunger (6) defines an internal air channel (11) communicating with the head (2) for evacuating the suction cup (30).

21. The extractor of Claim 19 or Claim 20, further including means (54, 68) for locking the plunger (6) with respect to the outer tube (8).

22. The extractor of Claim 21, wherein the plunger (6) includes an array of longitudinally spaced male locking formations (68) selectively engageable with female locking formations (54) on the outer tube (8).

23. The extractor of Claim 22, wherein the selected locking formations are engageable by turning the plunger (6) within the outer tube (8).

24. The extractor of any of Claims 16 to 23, further including suction means (36) communicating with the head (2) for evacuating the suction cup (30).

25. The extractor of any of Claims 21 to 24 when appendant to Claim 20, wherein the suction means (36) communicates with the head (2) via the internal air channel (11) of the plunger (6).

26. The extractor of Claim 24 or Claim 25, wherein the suction means comprises a syringe (36).

27. The extractor of Claim 26, wherein the syringe (36) has a piston (42, 70) and a one-way valve (44, 74) that opens to permit distal movement of the piston while closing upon proximal movement of the piston to draw air from the head (2).

28. The extractor of Claim 26 or Claim 27 when appendant to Claim 19, wherein the syringe (36) has a barrel (44) that serves as the plunger (6) of the actuator (6, 8).

29. The extractor of Claim 28, wherein the syringe (36) has an inner plunger (46) carrying the piston (70) and further includes means (58, 82) for locking the inner plunger with respect to the barrel (44).

30. The extractor of Claim 29, wherein the inner plunger (46) includes an array of longitudinally spaced male locking formations (82) selectively engageable with female locking formations (58) on the barrel (44).

31. The extractor of Claim 30, wherein the selected locking formations (58, 82) are engageable by turning the inner plunger (46) within the barrel (44).

32. The extractor of any of Claims 27 to 31, further including a reservoir (88) disposed distally with respect to the piston (70).

33. The extractor of any of Claims 24 to 32, further including a suction indicator (64) for indicating the degree of suction applied to the head (2).

34. The extractor of Claim 33, wherein the suction indicator comprises a diaphragm (64) responsive by deflection to the suction applied by the suction means (36).

35. The extractor of Claim 34 when appendant to Claim 32, wherein the reservoir (88) is disposed distally with respect to the diaphragm (64).

## Patentansprüche

1. Kopf (2) für einen Geburtshilfe-Vakuumextraktor (1), wobei der Kopf einen konvexen distalen Teil aufweist, **dadurch gekennzeichnet, dass**:
der Kopf von den Fingern eines Arztes zum Einfügen in eine Scheide einer Mutter seitlich zusammendrückbar ist; und
die Form des konvexen distalen Teils nach dem Einfügen zu einer konkaven Saugglocke (30) für den Eingriff mit einem Kopf eines Säuglings umgestaltbar ist, wodurch über die Glocke Kraft auf den Kopf des Säuglings ausgeübt werden kann, um die Entbindung des Säuglings zu unterstützen.

2. Kopf nach Anspruch 1, umfassend einen hohlen biegsamen Körper (2).

3. Kopf nach Anspruch 2, wobei die Wanddicke des hohlen Körpers proximal zunimmt.

4. Kopf nach Anspruch 2 oder Anspruch 3, umfassend eine relativ steife distale Glocke (22).

5. Kopf nach Anspruch 4, wobei die Glocke (22) konvex ist.

6. Kopf nach Anspruch 4 oder Anspruch 5, wobei mehrere verteilte Luftkanäle um die Glocke (22) herum angeordnet sind.

7. Kopf nach einem der Ansprüche 4 bis 6, wobei die Glocke (22) von dem Körper (2) beabstandet ist, um zuzulassen, dass Luft unter die Glocke strömt.

8. Kopf nach einem der Ansprüche 4 bis 7, wobei die Glocke (22) über dem distalen Ende des Körpers (2) liegt.

9. Kopf nach einem der vorangehenden Ansprüche, umfassend mindestens einen Luftabsaugkanal (18) zum Evakuieren der Saugglocke (30).

10. Kopf nach Anspruch 9, wenn zugehörig zu Anspruch 2, wobei der Luftabsaugkanal (18) das Innere des hohlen Körpers (2) umfasst.

11. Kopf nach Anspruch 9 oder Anspruch 10, wenn zugehörig zu Anspruch 6, wobei der Luftabsaugkanal (18) mit den durch die Glocke (22) definierten Luftkanälen in Verbindung steht.

12. Kopf nach Anspruch 11, wobei der Luftabsaugkanal (18) eine distale Öffnung des hohlen Körpers (2) unter der Glocke (22) umfasst.

13. Kopf nach einem der vorangehenden Ansprüche, umfassend Umgestaltungsmittel (6, 26) zum Wirken auf den distalen Teil, um die Glocke (30) zu definieren.

14. Kopf nach Anspruch 13, umfassend ein Zugmittel (26), das sich von dem distalen Ende des distalen Teils zu einer gegenüber dem Kopf (2) proximal befindlichen Betätigungsvorrichtung (6) erstreckt.

15. Kopf nach Anspruch 14, wenn zugehörig zu einem der Ansprüche 4 bis 8, wobei das distale Ende des Zugmittels (26) an der Glocke (22) verankert ist.

16. Geburtshilfe-Vakuumextraktor (1) mit einem Kopf (2) nach einem der vorangehenden Ansprüche.

17. Extraktor nach Anspruch 16, weiter umfassend Mittel (6, 26) zum Umgestalten oder Verformen des distalen Teils des Kopfs (2).

18. Extraktor nach Anspruch 17, wobei eine Betätigungsvorrichtung (6, 8) in einem proximalen Griffteil auf ein Zugmittel (26) wirkt, das sich distal zu dem Kopf (2) erstreckt.

19. Extraktor nach Anspruch 18, wobei die Betätigungsvorrichtung (6, 8) einen Stempel (6) umfasst, der gegenüber einer einen Griff definierenden äußeren Röhre (8) proximal bewegbar ist.

20. Extraktor nach Anspruch 19, wobei der Stempel (6) einen inneren Luftkanal (11) definiert, der mit dem Kopf (2) in Verbindung steht, um die Saugglocke (30) zu evakuieren.

21. Extraktor nach Anspruch 19 oder Anspruch 20, weiter umfassend Mittel (54, 68) zum Arretieren des Stempels (6) gegenüber der äußeren Röhre (8).

22. Extraktor nach Anspruch 21, wobei der Stempel (6) eine Anordnung von in Längsrichtung beabstandeten vorstehenden Arretierungsgebilden (68) umfasst, die selektiv mit zurückgesetzten Arretierungsgebilden (54) an der äußeren Röhre (8) in Eingriff treten können.

23. Extraktor nach Anspruch 22, wobei die ausgewählten Arretierungsgebilde in Eingriff treten können, indem der Stempel (6) in der äußeren Röhre (8) gedreht wird.

24. Extraktor nach einem der Ansprüche 16 bis 23, weiter umfassend ein Saugmittel (36), das mit dem Kopf (2) in Verbindung steht, um die Saugglocke (30) zu evakuieren.

25. Extraktor nach einem der Ansprüche 21 bis 24 wenn zugehörig zu Anspruch 20, wobei das Saugmittel (36) über den inneren Luftkanal (11) des Stempels (6) mit dem Kopf (2) in Verbindung steht.

26. Extraktor nach Anspruch 24 oder Anspruch 25, wobei das Saugmittel eine Spritze (36) umfasst.

27. Extraktor nach Anspruch 26, wobei die Spritze (36) einen Kolben (42, 70) und ein Einwegventil (44, 74) aufweist, das öffnet, um distale Bewegung des Kolbens zuzulassen und bei proximaler Bewegung des Kolbens schließt, um Luft aus dem Kopf (2) zu saugen.

28. Extraktor nach Anspruch 26 oder Anspruch 27 wenn zugehörig zu Anspruch 19, wobei die Spritze (36) einen Zylinder (44) aufweist, der als der Stempel (6) der Betätigungsvorrichtung (6, 8) dient.

29. Extraktor nach Anspruch 28, wobei die Spritze (36) einen inneren Stempel (46) aufweist, der den Kolben (70) trägt und weiter Mittel (58, 82) zum Verriegeln des inneren Stempels gegenüber dem Zylinder (44) umfasst.

30. Extraktor nach Anspruch 29, wobei der innere Kolben (46) eine Anordnung von in Längsrichtung beabstandeten vorstehenden Arretierungsgebilden (82) umfasst, die selektiv mit zurückgesetzten Arretierungsgebilden (58) am Zylinder (44) in Eingriff treten können.

31. Extraktor nach Anspruch 30, wobei die ausgewählten Arretierungsgebilde (58, 82) in Eingriff treten können, indem der innere Stempel (46) in dem Zylinder (44) gedreht wird.

32. Extraktor nach einem der Ansprüche 27 bis 31, weiter umfassend einen Behälter (88), der gegenüber dem Kolben (70) distal angeordnet ist.

33. Extraktor nach einem der Ansprüche 24 bis 32, weiter umfassend eine Soganzeige (64) zum Anzeigen des auf den Kopf (2) ausgeübten Maßes an Sog.

34. Extraktor nach Anspruch 33, wobei die Soganzeige eine Membran (64) umfasst, die durch Auslenkung auf den von dem Saugmittel (36) ausgeübten Sog reagiert.

35. Extraktor nach Anspruch 34, wenn zugehörig zu Anspruch 32, wobei der Behälter (88) gegenüber der Membran (64) distal angeordnet ist.

## Revendications

1. Tête (2) destinée à une ventouse (1) obstétrique, la tête présentant une partie distale convexe, **caractérisée en ce que** :
la tête est compressible latéralement par les doigts d'un physicien pour être introduite dans un vagin maternel ; et
la forme de la partie distale convexe est reconfigurable, après avoir été introduite, en une cupule (30) de succion concave pour se mettre en prise avec la tête d'un nouveau-né, ce qui permet d'appliquer une force sur la tête du nouveau-né par l'intermédiaire de la cupule pour aider à délivrer le nouveau-né.

2. Tête selon la revendication 1, comportant un corps (2) creux souple.

3. Tête selon la revendication 2, dans laquelle l'épaisseur de paroi du corps creux augmente dans la direction proximale.

4. Tête selon la revendication 2 ou la revendication 3, comportant un capuchon (22) distal relativement rigide.

5. Tête selon la revendication 4, dans laquelle le capuchon (22) est convexe.

6. Tête selon la revendication 4 ou la revendication 5, dans laquelle une pluralité de canaux d'air répartis sont disposés autour du capuchon (22).

7. Tête selon l'une quelconque des revendications 4 à 6, dans laquelle le capuchon (22) est espacé du corps (2) pour permettre à l'air de s'écouler sous le capuchon.

8. Tête selon l'une quelconque des revendications 4 à 7, dans laquelle le capuchon (22) surmonte l'extrémité distale du corps (2).

9. Tête selon l'une quelconque des revendications précédentes, comportant au moins un canal (18) d'extraction d'air pour évacuer la cupule (30) de succion.

10. Tête selon la revendication 9 lorsqu'elle est annexée à la revendication 2, dans laquelle le canal (18) d'extraction d'air comprend l'intérieur du corps (2) creux.

11. Tête selon la revendication 9 ou la revendication 10 lorsqu'elle est annexée à la revendication 6, dans laquelle le canal (18) d'extraction d'air communique avec les canaux d'air délimités par le capuchon (22).

12. Tête selon la revendication 11, dans laquelle le canal (18) d'extraction d'air comprend une ouverture distale du corps (2) creux sous le capuchon (22).

13. Tête selon l'une quelconque des revendications précédentes, comprenant un moyen (6, 26) de reconfiguration pour agir sur la partie distale afin de délimiter le capuchon (30).

14. Tête selon la revendication 13, comportant un moyen (26) de traction s'étendant de l'extrémité distale de la partie distale à un actionneur (6) dont la position est proximale par rapport à la tête (2).

15. Tête selon la revendication 14 lorsqu'elle est annexée à l'une quelconque des revendications 4 à 8, dans laquelle l'extrémité distale du moyen (26) de traction est ancrée au capuchon (22).

16. Ventouse (1) obstétrique pourvue d'une tête (2) selon l'une quelconque des revendications précédentes.

17. Ventouse selon la revendication 16, comprenant en outre un moyen (6, 26) pour reconfigurer ou déformer la partie distale de la tête (2).

18. Ventouse selon la revendication 17, dans laquelle un actionneur (6, 8) dans une partie de manche proximale agit sur le moyen (26) de traction s'étendant dans la direction distale par rapport à la tête (2).

19. Ventouse selon la revendication 18, dans laquelle l'actionneur (6, 8) comporte un plongeur (6) mobile dans la direction proximale par rapport à un tube (8) externe délimitant une poignée de manche.

20. Ventouse selon la revendication 19, dans laquelle le plongeur (6) délimite un canal (11) d'air interne communiquant avec la tête (2) pour évacuer la cupule (30) de succion.

21. Ventouse selon la revendication 19 ou la revendication 20, comprenant en outre un moyen (54, 68) de verrouillage du plongeur (6) par rapport au tube (8) externe.

22. Ventouse selon la revendication 21, dans laquelle le plongeur (6) comprend un réseau de saillies (68) de verrouillage mâles espacées longitudinalement pouvant se mettre en prise sélectivement avec des saillies (54) de verrouillage femelles sur le tube (8) externe.

23. Ventouse selon la revendication 22, dans laquelle les saillies de verrouillage choisies peuvent se mettre en prise en faisant tourner le plongeur (6) à l'intérieur du tube (8) externe.

24. Ventouse selon l'une quelconque des revendications 16 à 23, comprenant en outre un moyen (36) de succion communiquant avec la tête (2) pour évacuer la cupule (30) de succion.

25. Ventouse selon l'une quelconque des revendications 21 à 24 lorsqu'elle est annexée à la revendication 20, dans laquelle le moyen (36) de succion communique avec la tête (2) par l'intermédiaire du canal (11) d'air interne du plongeur (6).

26. Ventouse selon la revendication 24 ou la revendication 25, dans laquelle le moyen de succion comporte une seringue (36).

27. Ventouse selon la revendication 26, dans laquelle la seringue (36) est pourvue d'un piston (42, 70) et d'un clapet de non-retour (44, 74) qui s'ouvre pour permettre au piston de se déplacer dans la direction distale lorsqu'il se ferme au moment du déplacement proximal du piston pour aspirer l'air de la tête (2).

28. Ventouse selon la revendication 26 ou la revendication 27 lorsqu'elle est annexée à la revendication 19, dans laquelle la seringue (36) est pourvue d'un cylindre (44) qui sert de plongeur (6) de l'actionneur (6, 8).

29. Ventouse selon la revendication 28, dans laquelle la seringue (36) est pourvue d'un plongeur (46) interne portant le piston (70) et comprend en outre un moyen (58, 82) de verrouillage du plongeur interne par rapport au cylindre (44).

30. Ventouse selon la revendication 29, dans laquelle le plongeur (46) interne comprend un réseau de saillies (82) de verrouillage mâles espacées longitudinalement qui peuvent se mettre en prise sélectivement avec des saillies (58) de verrouillage femelles sur le cylindre (44).

31. Ventouse selon la revendication 30, dans laquelle les saillies (58, 82) de verrouillage choisies peuvent se mettre en prise en faisant tourner le plongeur (46) interne à l'intérieur du cylindre (44).

32. Ventouse selon l'une quelconque des revendications 27 à 31, comprenant en outre un réservoir (88) disposé dans la direction distale par rapport au piston (70).

33. Ventouse selon l'une quelconque des revendications précédentes 24 à 32, comprenant en outre un indicateur (64) de succion pour indiquer le degré de succion appliquée sur la tête (2).

34. Ventouse selon la revendication 33, dans laquelle l'indicateur de succion comporte une membrane (64) sensible par déflexion à la succion appliquée par le moyen (36) de succion.

35. Ventouse selon la revendication 34 lorsqu'elle est annexée à la revendication 32, dans laquelle le réservoir (88) est disposé dans la direction distale par rapport à la membrane (64).
